# EUROPEAN PATENT APPLICATION

(11) **EP 4 835 475 A1**
(43) Date of publication of application: **04.11.2026**
(21) Application number: 24912760.6
(22) Date of filing: 23.12.2024
(51) Int. Cl.: G01N 33/543

(54) **METHOD FOR DETECTING SUBSTANCE BEING TESTED FOR, AND LABELING BUFFER AND KIT USED THEREIN**

(30) Priority: 27.12.2023 JP 2023220891
(71) Applicant: Fujirebio Inc., Tokyo 107-0052 (JP)
(72) Inventor: SEKIYA, Shunsuke, Tokyo 107-0052 (JP); TANAKA, Miki, Tokyo 107-0052 (JP); ISHII, Yuichi, Tokyo 107-0052 (JP)
(74) Representative: Müller-Boré & Partner Patentanwälte PartG mbB
(86) International application number: PCT/JP2024/045549
(87) International publication number: WO 2025/142873

(57) **Abstract**

A method for immunologically detecting a test substance in a sample, comprising:
a labeling step of forming a complex of the test substance and a labeling body which contains a first probe molecule capable of binding to the test substance and a labeling substance, in the presence of at least one selected from imidazole and a derivative thereof, a pH buffering agent, and a polyvalent metal ion.

## Description

### [Technical Field]

The present invention relates to a method for detecting a test substance, as well as a labeling buffer and a kit for use in the same.

### [Background Art]

As a method for detecting a test substance in a sample, conventionally, immunological detection methods (immunoassay) utilizing a specific immune reaction between an antigen and an antibody have been widely used. In immunological detection methods mainly used today, for example, a target test substance is detected by labeling, with a labeling substance, a complex (antigen-antibody complex) formed by binding an antigen or an antibody (probe molecule) which is capable of specifically binding to the test substance, and detecting a signal derived from the labeling substance.

Such immunological detection methods include a non-competitive method which detects a test substance by binding, to a complex of a test substance and an antibody or an antigen which is capable of specifically binding to the test substance, an antibody or an antigen labeled with a labeling substance (labeling body), and a competitive method which detects a test substance by competitively binding, to a certain amount of an antibody or an antigen, a labeling body obtained by labeling the antigen or the antibody and the test substance. In addition, the immunological detection methods are also broadly divided into a homogeneous detection method which is conducted in a liquid state and a heterogeneous detection method which is conducted by using an immobilized antibody or the like.

For example, the non-competitive method includes a sandwich method which captures the test substance by using a capturing body in which an antibody or an antigen is immobilized on an insoluble carrier, binding an antibody or an antigen labeled with a labeling substance (labeling body) to this, and detecting the test substance by detecting a signal such as luminescence according to the labeling substance. As an example of such a sandwich method, International Publication No. WO2014/024853 (WO 2014/024853 A) (PTL 1) describes a sandwich method using a mixture of an anti-F1 antibody that specifically binds to prothrombin fragment 1 or an antigen-binding fragment thereof and an anti-F2 antibody that specifically binds to prothrombin fragment 2 or an antigen-binding fragment thereof as a labeled antibody (labeling body); and an anti-PIVKA-II antibody that specifically binds to PIVKA-II or an antigen-binding fragment thereof as an immobilized antibody (capturing body). Although various studies have been conducted on immunological detection methods so far in terms of a detection sensitivity, a detection accuracy, a reduction in non-specific reaction, and the like, further improvement has been required along with a reduction in amounts of samples, diversification of test substances, and the like, and particularly, further improvement in detection sensitivity has been demanded.

### [Citation List]

### [Patent Literature]

[PTL 1] International Publication No. WO2014/024853

### [Summary of Invention]

### [Technical Problem]

The present invention has been made in view of the above-described problems, and an object of the present invention is to provide a detection method capable of detecting a test substance with high sensitivity by an immunological detection method, as well as a labeling buffer for used in the detection method, and a kit comprising the same.

### [Solution to Problem]

As a result of earnestly studying a method for detecting a test substance by an immunological detection method, the present inventors found that by causing at least one selected from imidazole and a derivative thereof, a pH buffering agent, and a polyvalent metal ion to be present in a reaction system for forming a complex between the test substance and a labeling body, the value of a signal amount derived from a labeling substance of the labeling body is increased, making it possible to detect the test substance with high sensitivity even when the concentration of the test substance is low, and thus completed the present invention.

Aspects of the present invention obtained by such findings are as described below.
[1] A method for immunologically detecting a test substance in a sample, comprising:
   a labeling step of forming a complex of the test substance and a labeling body which contains a first probe molecule capable of binding to the test substance and a labeling substance in the presence of at least one selected from imidazole and a derivative thereof, a pH buffering agent, and a polyvalent metal ion.
[2] The detection method according to [1], wherein a concentration of the at least one selected from imidazole and a derivative thereof is 1 mM or more in a reaction system of the labeling step in terms of imidazole.
[3] The detection method according to [1] or [2], wherein a pH of a reaction system of the labeling step is 6.0 to 8.0.
[4] The detection method according to any one of [1] to [3], wherein a concentration of the polyvalent metal ion is 0.01 mM or more in a reaction system of the labeling step.
[5] The detection method according to any one of [1] to [4], further comprising a capturing step of capturing the test substance with a capturing body which contains a second probe molecule capable of binding to the test substance and an insoluble carrier.
[6] The detection method according to [5], further comprising a washing step of removing a contaminant which has not been captured by the capturing body after the capturing step.
[7] The detection method according to any one of [1] to [6], wherein the labeling substance is an enzyme, and the first probe molecule is an antibody capable of binding to the test substance.
[8] A labeling buffer for use in the detection method according to any one of [1] to [7], comprising at least one selected from imidazole and a derivative thereof, a pH buffering agent, and a polyvalent metal ion.
[9] The buffer according to [8], further comprising a labeling body which contains a first probe molecule capable of binding to the test substance and a labeling substance.
[10] A kit for use in the detection method according to any one of [1] to [7], comprising a labeling buffer which contains at least one selected from imidazole and a derivative thereof, a pH buffering agent, and a polyvalent metal ion.
[11] The kit according to [10], further comprising a labeling body which contains a first probe molecule capable of binding to the test substance and a labeling substance.

### [Advantageous Effects of Invention]

The present invention makes it possible to provide a detection method capable of detecting a test substance with high sensitivity by an immunological detection method, as well as a labeling buffer for use in the detection method, and a kit comprising the same.

### [Description of Embodiments]

Hereinafter, the present invention will be described in detail in accordance with the preferred embodiments.

### <Detection Method>

A detection method of the present invention is a method for immunologically detecting a test substance in a sample, comprising:
a labeling step of forming a complex of the test substance and a labeling body which contains a first probe molecule capable of binding to the test substance and a labeling substance in the presence of at least one selected from imidazole and a derivative thereof, a pH buffering agent, and a polyvalent metal ion.

### [Test Substance]

The "test substance" according to the present invention is a substance being tested for and is not particularly limited as long as in a relation with the first probe molecule and the second probe molecule as necessary (in the present Specification, collectively referred to simply as a "probe molecule" in some cases) which are described below, the probe molecule is capable of binding to, and preferably capable of specifically binding to the test substance. Such a combination of the test substance and the probe molecule (or a combination of the probe molecule and the test substance) includes combinations capable of achieving specific binding, such as for example, a combination of an antibody and an antigen, a combination of a lectin and a glycan capable of binding to the lectin (lectin-binding glycan), a combination of a receptor and a ligand, a combination of avidin and biotin, and a combination of an antibody containing an Fc region and an Fc-binding protein. Among these, in the present invention, it is preferable that the probe molecule be an antibody, and the test substance be a substance that can be an antigen to the antibody.

The test substance according to the present invention, for example, as a substance that can be an antigen to the antibody, includes antigen peptides, antibodies, receptor proteins, transport proteins, transcriptional regulators, haptens, enzymes, viral antigens·antibodies, and other proteins and peptides; saccharides (polysaccharides and monosaccharides); glycoproteins; nucleic acids; lipids; glycolipids; low-molecular-weight compounds such as pharmaceutical agents and the like including vitamins, hormones, coenzymes, toxins, antibiotics, immunosuppressants (for example, cyclosporin, tacrolimus, everolimus, sirolimus, methotrexate, and the like), and antiepileptic agents, but is not limited to these.

It is noted that in the present invention, the "antibody" encompasses not only full antibodies but also antibody fragments (for example, Fab, Fab', F(ab')₂, Fv, single-chain antibodies, diabodies, and the like) and low molecular-weight antibodies in which a variable region of an antibody is bound, and the "antibody" according to the present invention may be a polyclonal antibody or a monoclonal antibody.

### [Samples]

The "sample" used in the detection method of the present invention is not particularly limited as long as the sample is a sample in which the test substance can be present, and for example, various living organisms (including cells, tissues, organs, and individuals) and extract fluids thereof; samples collected from humans and animals other than humans (including body fluids such as saliva, oral mucosa, pharyngeal mucosa, tears, sweat, urine, induced sputum, bronchoalveolar lavage fluid, intestinal mucosa, serum, plasma, whole blood, spinal fluid, lymph, semen, ascitic fluid, and amniotic fluid; feces; and tissues) ; plant biological fluids; biological culture fluids; environmental water (rivers, lakes, harbors, waterways, groundwater, purified water, sewage, wastewater, and the like), suspension of solids (soils and the like), and the like can be used as appropriate in accordance with an object. The animals other than humans include, for example, primates such as chimpanzees and monkeys; livestock, poultry, and pets such as cows, pigs, horses, sheep, rabbits, chickens, cats, and dogs; wild animals and wild birds such as deer, boars, raccoons, weasels, mice, and pigeons.

Among these, for example, in the medical field or the clinical trial field, in the case of detecting, as a test substance, a biomarker or the like as a reference of a diagnosis of a disease, the sample generally includes samples collected from subjects (preferably, humans) such as a diagnostic subject for which to detect a target biomarker or the like, for example, serum, plasma, whole blood, urine, feces, oral mucosa, pharyngeal mucosa, intestinal mucosa, various biopsy tissues, and the like.

The sample may be a sample subjected to treatment such as pulverization or freezing, or may be a sample diluted or suspended with a diluent, or may be a sample adjusted in pH. The diluent includes, for example, water, saline, publicly-known buffer solutions (a sodium phosphate buffer, a MES buffer, a Tris buffer, a CFB buffer, a MOPS buffer, a PIPES buffer, a HEPES buffer, a tricine buffer, a bicine buffer, a glycine buffer, a citric acid buffer, a phosphoric acid buffer, and the like), organic solvents (dimethyl sulfoxide and the like), and may be those to which a stabilizing protein such as BSA, serum, or the like is added.

The sample to be subjected to the detection method of the present invention is preferably an aqueous sample, and is preferably diluted or suspended as necessary and appropriate. In addition, in the case where the test substance is a nucleic acid, or in the case where the test substance is a substance or the like derived from a microorganism contained in the sample, the sample may be a sample obtained by isolating these nucleic acid and microorganism as appropriate. As the method for isolating such nucleic acid or microorganism from the sample, a publicly-known method can be employed as appropriate.

### [Labeling Body]

In the present invention, the "labeling body" is a complex which contains a labeling substance and a first probe molecule capable of binding to the test substance, and is a conjugate in which the labeling substance and the first probe molecule are directly or indirectly bound to each other. In addition, the labeling body may further contain a water-soluble carrier or the like which supports the labeling substance and the first probe molecule.

### (Labeling Substance)

The "labeling substance" contained in the labeling body according to the present invention mainly functions as a label for detecting a test substance, and a labeling substance used as a labeling substance in a publicly-known immunological detection method can be employed as appropriate, but is preferably an enzyme in the present invention from the viewpoint that particularly the detection sensitivity is improved by the detection method of the present invention.

The enzyme includes, for example, alkaline phosphatase (ALP), horseradish peroxidase (HRP), β galactosidase (β-gal), glucose oxidase, and luciferase, and may be one of these or a combination of two or more of these. In the case where an enzyme is used as the labeling substance, by adding a chromogenic substrate, a fluorescent substrate, a chemiluminescent substrate, or the like as a substrate, the following signal according to the substrate can be detected. As such labeling substance and substrate, conventionally known ones can be used as appropriate, and commercially-available ones can also be used.

### (First Probe Molecule)

In the present invention, the "first probe molecule" indicates a molecule capable to binding to, and preferably capable of specifically binding to the test substance. In the case where a capturing body described below is used, and a capturing step described below is carried out before the labeling step or simultaneously with the labeling step, the "first probe molecule capable of binding to the test substance" encompasses a probe molecule capable of binding to, and preferably capable of specifically binding to a complex of the test substance and the second probe molecule. A form of binding to a complex of the test substance and the second probe molecule includes, for example, a form of recognizing and binding to a binding site of the test substance and the second probe molecule.

The first probe molecule according to the present invention only has to be capable of binding to, and preferably capable of specifically binding to the test substance in a relation with the test substance, may be one or a combination of two or more, but is preferably an antibody to the test substance correspondingly. In addition, it is more preferable that the labeling body according to the present invention be an enzyme-labeled antibody in which the labeling substance is an enzyme and the first probe molecule is an antibody. Such a first probe molecule can be prepared by a publicly-known established method in accordance with the test substance, and a commercially-available one may be used as appropriate.

### (Configuration and Production Method of Labeling Body)

In the labeling body according to the present invention, a molar ratio between the labeling substance and the first probe molecule is not particularly limited, and can be adjusted as appropriate in accordance with a combination of these types, easiness in binding to the test substance, and the like. For example, the first probe molecule (in the case where the first probe molecule is a combination of two or more, the total of these) is preferably 0.01 to 10000 mol, and more preferably 0.05 to 10 mol, relative to 1 mol of the labeling substance (in the case where the labeling substance is a combination of two or more, the total of these).

The labeling body according to the present invention can be produced by binding the labeling substance and the first probe molecule. As this production method, a conventionally known method or a method compliant with the conventionally known method can be employed as appropriate in accordance with the types of the labeling substance and the first probe molecule, and the labeling substance and the first probe molecule may be directly bound or indirectly bound to each other.

The directly binding method includes, for example, a method which adds an active group (for example, a thiol group, a maleimide group, or a succinimide group) to the labeling substance and/or the first probe molecule, or uses those having these active groups as the labeling substance and/or the first probe molecule, and binds these through covalent bonding by the active group. As the labeling substance and the first probe molecule to which the active group has been added, commercially-available ones may be used as they are, or the labeling substance and the first probe molecule may be prepared by introducing the active group into the surface of the labeling substance and/or the first probe molecule under appropriate reaction conditions. In addition, the method for indirectly binding the labeling substance to the first probe molecule includes, for example, a method which binds the labeling substance to the first probe molecule via polyhistidine, polyethylene glycol, an oligopeptide, a linker molecule, or the like. In addition, one of these may be modified in a certain fashion while a substance which captures the modified portion is added to the other, and the labeling substance and the first probe molecule are bound via these. For example, a binding method in which one of these is biotinylated while the other is avidinated, and these are bound via an avidin-biotin bond. A ratio between the labeling substance and the first probe molecule to be subjected to this production method can be selected as appropriate to achieve the above-described preferable range in the labeling body. In addition, as such a labeling body, for example, a commercially-available one such as an enzyme-labeled antibody may also be used as appropriate.

### [Capturing Body]

In the production method of the present invention, a capturing body is preferably used. In the present invention, the "capturing body" is a complex containing an insoluble carrier and a second probe molecule capable of binding to the test substance, and is a conjugate in which the second probe molecule is directly or indirectly bound to and is supported on the insoluble carrier.

### (Insoluble Carrier)

The "insoluble carrier" contained in the capturing body according to the present invention is water insoluble, and functions as a carrier which supports and immobilizes mainly the second probe molecule. It is noted that in the present invention, the term "water insoluble" indicates that the carrier is insoluble in water at ordinary temperature and ordinary pressure (the solubility to water is 0.001 g/mL or less, and preferably 0.0001 g/mL or less, the same applies hereinbelow).

As the material of such an insoluble carrier, a material used as an insoluble carrier in a publicly-known immunological detection method can be used as appropriate, and is not particularly limited. The material includes, for example, at least one selected from the group consisting of high molecular polymers (polystyrene, (meth)acrylic acid ester, polymethyl methacrylate, polyimide, nylon, and the like), gelatin, cellulose, nitrocellulose, glass, latex, silica, metals (gold, platinum, and the like), and metal compounds (iron oxide, ferrite, cobalt oxide, nickel ferrite, and the like). In addition, the material of the insoluble carrier may be a composite material of these, and may be, for example, an organic-inorganic composite material composed of at least one organic polymer from the group consisting of the high molecular polymer, gelatin, cellulose, and latex and at least one metal compound from the group consisting of iron oxide (spinel ferrite or the like), cobalt oxide, and nickel ferrite.

In addition, in the present invention, the shape of the insoluble carrier is not particularly limited, and may be, for example, any of a plate, fibers, a film, particles, and the like, but is preferably particles from the viewpoint of reaction efficiency, and more preferably magnetic particles from the viewpoint of automation·reduction in time. As such an insoluble carrier, a conventionally known one can be used as appropriate, and a commercially-available one can also be used.

### (Second Probe Molecule)

In the present invention, the "second probe molecule" indicates a molecule capable of binding to, and preferably capable of specifically binding to the test substance. In the case where the capturing step described below is carried out after the labeling step or simultaneously with the labeling step, the "second probe molecule capable of binding to the test substance" encompasses a probe molecule capable of binding to, and preferably capable of specifically binding to a complex of the test substance and the first probe molecule. A form of binding to a complex of the test substance and the first probe molecule includes, for example, a form of recognizing a binding site of the test substance and the first probe molecule.

The second probe molecule according to the present invention only has to be capable of binding to, and preferably specifically binding to the test substance in a relation with the test substance, may be one or a combination of two or more, but is preferably an antibody to the test substance correspondingly. In addition, the second probe molecule may be different from the first probe molecule, or may be the same as the first probe molecule as long as the second probe molecule does not inhibit the effects of the present invention. Such a second probe molecule can be prepared by a publicly-known established method in accordance with the test substance, and a commercially-available one may be used as appropriate.

### (Configuration and Production Method of Capturing Body)

In the capturing body according to the present invention, a content of the second probe molecule is not particularly limited, but is preferably set such that the number of molecules of the second probe molecule binding to one molecule of the insoluble carrier becomes as large as possible in order to further improve the detectability of the test substance. For example, a mass of the second probe molecule (in the case where the second probe molecule is a combination of two or more, the total of these) is preferably 0.005 to 0.05 parts by mass, and more preferably 0.01 to 0.04 parts by mass, relative to 100 parts by mass of the mass of the insoluble carrier.

The capturing body according to the present invention can be produced by binding and immobilizing the second probe molecule to the insoluble carrier. As this production method, a conventionally known method or a method compliant with the conventionally known method can be employed as appropriate in accordance with the types of the insoluble carrier and the second probe molecule. The second probe molecule may be directly bound to the insoluble carrier, or may be indirectly bound to the insoluble carrier. Such a binding method includes a method presented as the method for binding the labeling substance and the first probe molecule. A ratio between the insoluble carrier and the second probe molecule to be subjected to this production method can be selected as appropriate to achieve the above-described preferable range of each content in the capturing body. In addition, as such a capturing body, for example, a commercially-available one such as antibody-binding particles may also be used as appropriate.

### [Labeling Step]

In the detection method of the present invention, in the labeling step, the sample and the labeling body are brought into contact with each other, and in the case where the test substance is present in the sample, a complex of the labeling body and the test substance (in the present Specification, referred to as a "first complex" in some cases), that is, a labeling body-test substance complex is formed via the binding between the test substance and the first probe molecule. Alternatively, in the case where the detection method of the present invention includes the capturing step described below before the labeling step or simultaneously with the labeling step, in the labeling step, a complex of the labeling body and the test substance captured by the capturing body, that is, a labeling body-test substance-capturing body complex (in the present Specification, referred to as a "second complex" in some cases) is formed.

The method for bringing the sample and the labeling body into contact with each other is not particularly limited, and a conventionally known method or a method compliant with the conventionally known method can be employed as appropriate, and includes, for example, a method for adding the labeling body to the aqueous sample.

In the labeling step according to the present invention, in the reaction system between the test substance and the labeling body, at least one selected from imidazole and a derivative thereof (in the present Specification, collectively referred to as "imidazole or the like" in some cases); a pH buffering agent; and a polyvalent metal ion are caused to be present. In the present invention, the "reaction system between the test substance and the labeling body" is water-based, and is preferably an aqueous solution containing the labeling body and the test substance for reacting these.

### (Imidazole or the Like)

In the present invention, the "imidazole and a derivative thereof" indicates a compound having an imidazole skeleton, and includes, for example, imidazole, 1-methylimidazole, 1-ethylimidazole, 1-propylimidazole, 1-butylimidazole, 1-phenylimidazole, 1-vinylimidazole, 1-allylimidazole, 1-benzyl-2-methylimidazole, 1-benzyl-2-formylimidazole, 1-benzyl-4-hydroxymethylimidazole, 1-benzyl-5-hydroxymethylimidazole, 1-(2-hydroxyethyl)-imidazole, 1-(2-hydroxyethyl)-2-methylimidazole, 2-methylimidazole, 2-ethylimidazole, 2-propylimidazole, 2-butylimidazole, 2-phenylimidazole, 2-formylimidazole, 2-hydroxymethylimidazole, 2-methyl-1-vinylimidazole, 2-butyl-4-formylimidazole, 2-butyl-4-hydroxymethylimidazole, 2-butyl-4-chloro-5-formylimidazole, 2-hydroxymethyl-1-benzylimidazole, 2-hydroxymethyl-2-methylimidazole, 2-ethyl-4-methylimidazole, 4-butylimidazole, 4-formylimidazole, 4-formyl-1-methylimidazole, 4-formyl-1-tritylimidazole, 5-formyl-1-methylimidazole, 4-formyl-5-methylimidazole, 4-hydroxymethylimidazole hydrochloride, methylimidazole-4-carboxylate, ethylimidazole-4-carboxylate, 1,2-dimethylimidazole, 1,2,4-trimethylimidazole, and may be one of these or a combination of two or more of these.

The imidazole or the like also encompasses chemically acceptable salts of the imidazole or a derivative thereof, and such salts include, for example, potassium salt, magnesium salt, lithium salt, calcium salt, zinc salt, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, sesqui(fumarate), hydrochlorate, dihydrochlorate, trihydrochlorate, hydrobromide, hydroiodide, 2-hydroxyethane sulfonate (isethionate), lactate, maleate, methanesulfonate, nicotinate, 2-naphthalenesulfonate, oxalate, pamoate, pectinate, persulfate, 3-phenylproprionate, picrate, pivalate, propionate, succinate, sulfate, bis(tartrate), tartrate, (L) tartrate, bis((L)tartrate), (D)tartrate, (DL)tartrate, bis((DL)tartrate), mesotartrate, bis (mesotartrate), thiocyanate, phosphate, glutamate, hydrocarbonate, bis((D)tartrate), bis(bromide), bis(sulfate), bis (phosphate), tris(hydrochlorate), p-toluenesulfonate, and undecanoate, and may be one of these or a combination of two or more of these. It is noted that among these, the imidazole or the like to be added to the reaction system is preferably a salt that does not generate the polyvalent metal ion, and is more preferably not a salt, from a viewpoint of controlling concentrations of the imidazole or the like and the polyvalent metal ion described below in the reaction system. However, it is not denied that the imidazole or the like and the polyvalent metal ion described below form a salt in the reaction system.

In the labeling step according to the present invention, a concentration of the imidazole or the like (in the case where the imidazole or the like is two or more, the total of these, the same applies hereinbelow) in the reaction system is preferably 1 mM or more, more preferably 1 to 30 mM, further preferably 2 to 28 mM, even more preferably 3 to 25 mM, and particularly preferably 5 to 15 mM, in terms of imidazole, that is, in terms of imidazole skeleton amount. If the concentration of the imidazole or the like is less than the lower limit, there is a tendency that the effect of improving the detection sensitivity is not sufficiently exhibited. On the other hand, if the concentration of the imidazole or the like is more than the upper limit, there is a case where the detection sensitivity decreases on the contrary.

### (pH Buffering Agent)

In the present invention, the "pH buffering agent" only has to have a pH-buffering ability (a function to suppress an abrupt change of the pH) for an aqueous solution, but it is preferable that the pH buffering agent according to the present invention be capable of maintaining the pH of the reaction system, which is an aqueous solution, at 6.0 to 8.0, and preferably 7.0 to 7.5.

Such a pH buffering agent includes, for example, lactic acid, acetic acid, hydrochloric acid, succinic acid, phthalic acid, phosphoric acid, boric acid, citric acid, maleic acid, Tris (tris(hydroxymethyl)aminomethane), Bis-Tris (bis(2-hydroxyethyl)iminotris(hydroxymethyl)methane), MES (2-morphholinoethanesulfonic acid), ADA (N-2(acetoamido)iminodiacetic acid), PIPES (piperazine-1,4-bis(2-ethanesulfonic acid)), ACES (N-(2-acetamido) -2-aminoethanesulfonic acid), BES (N,N-bis(2-hydroxyethyl)-2-aminoethanesulfonic acid), TES (N-tris(hydroxymethyl)methyl-2-aminoethanesulfonic acid), HEPES (2-[4-(2-hydroxyethyl)-1-piperazinyl]-ethanesulfonic acid), MOPS (3-morpholinopropane-1-sulfonic acid), Tricine (N-[tris(hydroxymethyl)methyl]glycine), and Bicine (N,N-bis(2-hydroxyethyl)glycine), and may be one of these or a combination of two or more of these. Among these, in the case where the labeling body is an enzyme-labeled antibody , Tris is preferable from the viewpoint that Tris does not inhibit the enzyme activity.

In the labeling step according to the present invention, a concentration of the pH buffering agent in the reaction system may be selected as appropriate in accordance with the type of the pH buffering agent to be used, but it is preferable that the concentration be such a concentration that the pH of the reaction liquid becomes 6.0 to 8.0, and preferably 7.0 to 7.5. For example, in the case of Tris, a specific concentration is preferably 10 to 500 mM, and more preferably 50 to 200 mM.

### (Polyvalent Metal Ion)

In the present invention, the "polyvalent metal ion" indicates a divalent or more metal ion, and includes, for example, divalent metal ions such as a zinc ion, a calcium ion, a magnesium ion, a copper ion, an iron(II) ion, a nickel ion, a manganese ion, and a cobalt ion; and trivalent metal ions such as an iron(III) ion, a chromium ion, and an aluminum ion, and may be one kind of these or a combination of two or more of these. Among these, the polyvalent metal ion according to the present invention is preferably a divalent metal ion, more preferably at least one selected from the group consisting of a zinc ion, a magnesium ion, and a nickel ion, further preferably a zinc ion and/or a nickel ion, and even more preferably a zinc ion.

These polyvalent metal ions may be added to the reaction system in the form of chemically acceptable ionic compounds of these. In this case, the form of the ionic compound includes a chloride, a sulfide, an iodide, and a hydroxide, and may be one of these or a combination of two or more of these. However, in the case where the labeling body is an enzyme-labeled antibody, a chloride is preferable from the viewpoint that the chloride does not inhibit the enzyme activity. In addition, in the case where the imidazole or the like to be added to the reaction system is in the form of a salt, and generates the polyvalent metal ion, this may also be used as the ionic compound, but it is preferable that the imidazole or the like to be added do not generate the polyvalent metal ion.

In the labeling step according to the present invention, a concentration of the polyvalent metal ion (in the case where the polyvalent metal ion is two or more kinds, the total of these, the same applies hereinbelow) in the reaction system is preferably 0.01 mM or more, more preferably 0.1 to 10 mM, further preferably 0.2 to 8 mM, even more preferably 0.3 to 5 mM, and particularly preferably 0.3 to 3 mM. If the concentration of the polyvalent metal ion is less than the lower limit, there is a tendency that the effect of improving the detection sensitivity is not sufficiently exhibited. On the other hand, if the concentration of the polyvalent metal ion is more than the upper limit, there is a tendency that the detection sensitivity decreases on the contrary or the metal ion precipitates.

### (Labeling Body)

In the labeling step according to the present invention, a concentration of the labeling body in the reaction system is adjusted as appropriate in accordance with the types, concentrations, and the like of the sample and the labeling body, and is thus not particularly limited. However, for example, in the case where the labeling body is an enzyme-labeled antibody, the concentration of the labeling body is preferably 0.1 to 5 µg/mL, and more preferably 0.5 to 2 µg/mL.

### (Others)

In the labeling step according to the present invention, the pH of the reaction system is preferably 6.0 to 8.0, and more preferably 7.0 to 7.5, as mentioned above. The temperature and reaction time of the reaction system are not particularly limited, and can be adjusted as appropriate. For example, the reaction can be conducted at room temperature to 45°C, and preferably 20 to 37°C, for around 5 seconds to 10 minutes, and preferably around 30 seconds to 8 minutes.

In the labeling step according to the present invention, the reaction system may further contain other components such as, for example, a salt, a saccharide, a protein, a surfactant, a preservative besides the test substance, the labeling body, the imidazole or the like, the pH buffering agent, and the polyvalent metal ion.

The salt indicates a salt to be added to the reaction system as a salt other than a salt of the above-described imidazole or the like, the above-described pH buffering agent, and a salt derived from an ionic compound of the above-described polyvalent metal ion, and includes, for example, sodium chloride and potassium chloride, and may be one of these or a combination of two or more of these. In the case where the reaction system contains the salt, a content of the salt (in the case where the salt is two or more, the total of these, the same applies hereinbelow) is, for example, preferably 50 to 1000 mM, and more preferably 50 to 300 mM.

The saccharide includes, for example, glucose, sucrose, maltose, sorbitol, mannitol, xylitol, trehalose, and cyclodextrin, and may be one of these or a combination of two or more of these. In the case where the reaction system contains the saccharide, a content of the saccharide (in the case where the saccharide is two or more, the total of these, the same applies hereinbelow) is, for example, preferably 0.5 to 50 w/v%, and more preferably 1 to 40 w/v%.

The protein includes, for example, casein, casein sodium, bovine serum albumin (BSA), and fetal bovine serum (FBS), and may be one of these or a combination of two or more of these. In the case where the reaction system contains the protein, a content of the protein (in the case where the protein is two or more, the total of these, the same applies hereinbelow) is, for example, preferably 0.01 to 10 w/v%, and more preferably 0.1 to 5 w/v%.

The surfactant includes, for example, a nonionic surfactant, a cationic surfactant, an anionic surfactant, and an ampholytic surfactant, and may be one of these or a combination of two or more of these, but is preferably a nonionic surfactant (for example, Tween 20, Tween 80 (produced by Merck KGaA) or the like). In the case where the reaction system contains the surfactant, a content of the surfactant (in the case where the surfactant is two or more, the total of these, the same applies hereinbelow) is, for example, preferably 0.01 to 5 w/v%, and more preferably 0.1 to 1 w/v%.

The preservative includes, for example, sodium azide, an antibiotic, and ProClin (produced by Merck KGaA), and may be one of these or a combination of two or more of these. In the case where the reaction system contains the preservative, a content of the preservative can be adjusted as appropriate in accordance with the type of the preservative.

In addition, besides, for example, in the case where the labeling body is an enzyme-labeled antibody, the other component includes an inactivated enzyme obtained by inactivating the same enzyme as this enzyme, and for example, in the case where the labeling body is an ALP-labeled antibody, the inactivated enzyme includes an inactivated ALP. This makes it possible to suppress non-specific reaction (see Japanese Unexamined Patent Application Publication No. 2008-281489 (JP 2008-281489 A)). In the case where the reaction system contains the inactivated enzyme, a content of the inactivated enzyme is preferably 10 to 5000 parts by mass relative to 1 parts by mass of the labeling body. In addition, besides, the other component also includes, for example, polymers such as PEG, Dextran, and Lipidure (produced by NOF Corporation); and amino acids.

Moreover, in the labeling step according to the present invention, the reaction system may further contain a component derived from the sample (for example, a contaminant other than the test substance contained in the sample). However, in the detection method of the present invention, it is preferable to carry out a capturing step and a washing step described below to remove such component before the labeling step, and then conduct the labeling step in the above-described reaction system. In addition, in the labeling step according to the present invention, the reaction system may further contain a component derived from the labeling body (for example, in the case where a storage buffer of the labeling body is other than a labeling buffer described below, this storage buffer (for example, those given as the diluent)); however, it is preferable to add the labeling body alone or in the form of a labeling buffer described below to the reaction system.

### [Capturing Step]

It is preferable that the detection method of the present invention comprise, before the labeling step, a capturing step of bringing the sample and the capturing body into contact with each other, and in the case where a test substance is present in the sample, capturing the test substance with the capturing body via the binding of the test substance and the second probe molecule to form a complex of the capturing body and the test substance, that is, a capturing body-test substance complex (in the present Specification, referred to as a "third complex" in some cases). Alternatively, it is also preferable that the detection method of the present invention comprise, after the labeling step or simultaneously with the labeling step, a capturing step of bringing a first complex obtained in the labeling step and the capturing body to form a second complex of labeling body-test substance-capturing body. It is more preferable that such a capturing step be included before the labeling step from the viewpoint of removing the above-described a contaminant other than the test substance contained in the sample and the viewpoint of further improving the detection accuracy by conducting a washing step multiple times.

The method for bringing the sample and the capturing body into contact with each other is not particularly limited, and a conventionally known method or a method compliant with the conventionally known method can be employed as appropriate. For example, the method includes, in the case where the insoluble carrier is a plate, a method that injects the sample into the insoluble carrier, and in the case where the insoluble carrier is particles, a method that mixes the particle solution containing the insoluble carrier and the sample. A dispersion medium for the particle solution includes, for example, those given as the diluent.

In the reaction between the capturing body and the test substance, a content (final concentration) of the capturing body in a reaction liquid containing the capturing body is not particularly limited, and is adjusted as appropriate in accordance with the types, concentrations, and the like of the sample and the capturing body, and is thus not particularly limited, but, for example, is preferably 0.75 to 75 µg/mL, and more preferably 5 to 30 µg/mL, in terms of an amount of the second probe molecule. In addition, the conditions of the capturing step are also not particularly limited and can be adjusted as appropriate, and the capturing step can be conducted, for example, at room temperature to 45°C, and preferably 20 to 37°C, at a pH of around 6 to 9, and preferably a pH of 7 to 8, for around 5 seconds to 10 minutes, and preferably around 30 seconds to 8 minutes, but the conditions are not limited to these conditions.

### [Washing Step]

In the case where the detection method of the present invention comprises the capturing step, it is preferable that the detection method further comprise a washing step of removing a contaminant which has not been captured by the capturing body. In the case where the capturing step is comprised before the labeling step, it is more preferable that the washing step be comprised between the capturing step and the labeling step, and a contaminant which has not been captured by the capturing body, that is, a component other than the third complex be removed. In addition, in this case, it is more preferable that a washing step be further comprised after the labeling step, and a contaminant which has not been captured by the capturing body, that is, a component other than the second complex contained in the reaction system be removed.

The method for removing a contaminant is not particularly limited, and a conventionally known method or a method compliant with the conventionally known method can be employed as appropriate. For example, the method includes, in the case where the insoluble carrier is a plate, a method that removes a liquid phase (supernatant) from on the plate, and in the case where the insoluble carrier is particles, a method that collects the particles from the reaction buffer by centrifugation or magnetic collection and removes a liquid phase (supernatant). In addition, in the washing step, subsequently, injection and removal of the washing solution may be repeated as necessary. The washing solution includes, for example, neutral (preferably, pH 6 to 9) publicly-known buffer solutions (such as sodium phosphate buffer, MES buffer, Tris buffer, CFB buffer, MOPS buffer, PIPES buffer, HEPES buffer, tricine buffer, bicine buffer, and glycine buffer), or may be a washing solution to which a stabilizing protein such as BSA, a surfactant, or the like is added.

### [Detecting Step]

In the detection method of the present invention, the test substance is indirectly detected by detecting a signal derived from the labeling substance of the labeling body which has bound to the test substance (detecting step). The "signal" includes, for example, fluorescence, luminescence, and color reaction (color production), and includes those which can be checked by the naked eye, and also those which can be checked by a fluorescence microscope and an electrical analysis. According to the detection method of the present invention, the signal amount generated from the reaction between one test substance and the labeling body can be increased by conducting the labeling step in the reaction system of the above-described conditions, and the detection sensitivity can be significantly increased even when the concentration of the test substance is low.

In the case where an enzyme is used as the labeling substance, preferably by carrying out the capturing step and the washing step before the labeling step and the washing step after the same labeling step to remove a contaminant which has not been captured by the capturing body, and adding and reacting a chromogenic substrate, a fluorescent substrate, a chemiluminescent substrate, or the like according to the type of the enzyme as a substrate, a signal (fluorescence, luminescence, or color reaction (color production)) according to the substrate can be detected. Such a substrate and reaction conditions can be adjusted as appropriate in accordance with the types of the enzyme and the like. As the detection method of the present invention, a value (count) of the detected signal amount may be used as a value corresponding to the amount of the test substance, and as necessary, may be compared with a value of signal amount in a reference sample in which the test substance has a known concentration to quantify the test substance.

### <Labeling Buffer, Kit>

The present invention provides a buffer comprising at least one selected from imidazole and a derivative thereof a pH buffering agent, and a polyvalent metal ion as a labeling buffer for use in the above-described detection method of the present invention.

The labeling buffer, including its preferable aspects, is as mentioned above as the reaction system of the labeling step, and the composition and the content of each component of the reaction system can be replaced with the composition and the content of each component of the labeling buffer, respectively. The labeling buffer according to the present invention is preferably an aqueous solution containing each component. In addition, the labeling buffer according to the present invention can be, for example, a liquid concentrate in which the content of each component is set to a 1 to 10 times concentration such that the labeling buffer can be diluted for use.

It is preferable that the labeling buffer further contain the labeling body. This labeling body, including its preferable aspects, is as mentioned above. The labeling buffer containing the labeling body can be distributed and stored as a labeling body fluid as it is, and for example, after the capturing step and the washing step are conducted on the sample, the labeling step according to the present invention can be carried out by adding the labeling buffer as it is or after dilution as necessary.

In addition, the present invention provides a kit comprising the labeling buffer as a kit for use in the above-described detection method of the present invention. It is preferable that the kit of the present invention further comprise the labeling body. In this case, the labeling buffer included in the kit may be a labeling buffer containing the labeling body, or the kit may separately include a labeling buffer containing no labeling body, and the labeling body. In addition, the kit of the present invention may further include the capturing body. This capturing body, including its preferable aspects, is also as mentioned above. These may be each independently a solid (powder) or may be a liquid obtained by dissolving or suspending these in the storage buffer.

The kit of the present invention may further include at least one selected from the group consisting of a reference sample (concentration), a control sample, the diluent, the washing solution, the substrate, an enzyme-substrate reaction buffer, and an enzyme-substrate reaction termination buffer, for example. In addition, the kit for the detection method of the present invention may further include instructions for the kit.

In addition, the present invention also provides a method for improving detection sensitivity of a method for immunologically detecting a test substance in a sample, comprising: a labeling step of forming a complex of the test substance and a labeling body which contains a first probe molecule capable of binding to the test substance and a labeling substance in the presence of at least one selected from imidazole and a derivative thereof, a pH buffering agent, and a polyvalent metal ion. It is preferable that the sensitivity improving method of the present invention further comprise a capturing step, a washing step, and/or a detecting step, and as the labeling step, the capturing step, the washing step, the detecting step, the sample, the test substance, the labeling body, the imidazole or the like (at least one selected from imidazole and a derivative thereof), the pH buffering agent, the polyvalent metal ion, and other components and conditions according to this sensitivity improving method, including their preferable aspects, each aspect described in the above-described detection method of the present invention can be applied.

### [Examples]

Hereinafter, the present invention will be described in more detail based on Examples and Comparative Examples; however, the present invention is not limited to the following Examples. It is noted that in each of Examples and Comparative Examples, the indication of "%" indicates weight/capacity (w/v: g/mL) percent unless otherwise specified.

### (1) Preparation of Antibody-binding Magnetic Particles

N-Hydroxysuccinimide (NHS) and 1-ethyl-3-(3-dimethylaminopropyl)carbodiimide Hydrochloride (EDC hydrochloride) were added to and reacted with magnetic particles (ferrite particles). After the particles thus reacted were washed, a first anti-cyclosporin antibody was added and reacted. To terminate the reaction, 1M Tris-HCl was added. Subsequently, after pre-masking was conducted with a 10% BSA solution, a masking buffer (100 mM NaHCO₃, 2.0% BSA, 1 mM EDTA, 0.1% NaN₃, pH 9.0) was reacted. This was dispersed into a storage buffer (50 mM Tris-HCl, 2.0% BSA, 150 mM NaCl, 10% sucrose, 0.1% ProClin 300, pH 7.2) to prepare a magnetic particle solution containing antibody-binding magnetic particles. As the first anti-cyclosporin antibody, a monoclonal antibody to cyclosporin was used.

### (2) Preparation of Alkaline Phosphatase (ALP)-Labeled Antibody

A second anti-cyclosporin antibody desalted with a thiolation buffer and iminothiolane were mixed to conduct thiolation. Subsequently, the mixture was desalted by using a coupling buffer (0.1M phosphoric acid buffer, 0.1% CHAPS, 1 mM EDTA, pH 7.0) to obtain a thiolated antibody. On the other hand, desalted alkaline phosphatase and N-(4-maleimidebutylyloxy)-succinimide (GMBS) were mixed in a 0.1M phosphoric acid buffer to obtain maleimidated ALP. Subsequently, after the maleimidated ALP was desalted by using a coupling buffer, this and the thiolated antibody were mixed to conduct coupling. After the coupling, L-cysteine was added, and further, iodoacetamide was added to block free thiol groups. This was concentrated, and gel filtration purification was conducted to obtain an ALP-labeled antibody. As the second anti-cyclosporin antibody, a monoclonal antibody obtained by expressing, from E. coli, a VHH antibody having an amino acid sequence binding to the binding site between cyclosporin and the first anti-cyclosporin antibody (obtained from the naïve library of alpaca) by a conventional method, followed by purification was used.

### (3) Preparation of Cyclosporin Solution

Cyclosporin was dissolved into dimethyl sulfoxide and prepared to be 10 mg/mL to obtain a primary stock. The primary stock was dissolved into a diluent for cyclosporin (phosphoric acid buffer-based diluent) and prepared to be 50 µg/mL to obtain a secondary stock. The secondary stock was added to the diluent for cyclosporin such that the concentration of cyclosporin became each concentration to prepare a cyclosporin solution.

### (Examples 1 to 3 and Comparative Example 1)

From the cyclosporin solution prepared in the above-described (3), cyclosporin was detected by using the antibody-binding magnetic particles and the ALP-labeled antibody prepared in the above-described (1) and (2). Specifically, first, 20 µL of the cyclosporin solution (concentration of cyclosporin: 0 to 2000 [ng/mL]) prepared in the above-described (3) and 100 µL of a citric acid buffer-based pretreatment liquid were mixed, and reacted at 37°C for 6.5 minutes. Subsequently, 50 µL of a magnetic particle solution containing the antibody-binding magnetic particles prepared in the above-described (1) was mixed, and reacted at 37°C for 8 minutes. The magnetic particles were magnetically collected and washed to remove a contaminant which did not bind to the magnetic particles. Subsequently, the ALP-labeled antibody prepared in the above-described (2) was added to 50 µL of a labeling body fluid diluted with a labeling buffer which did not contain imidazole or contained 5 to 20 mM of imidazole (a concentration of the labeling body in the reaction system: 2 µg/mL), and reacted at 37°C for 8 minutes. After the reaction, the magnetic particles were magnetically collected and washed to remove a contaminant which did not bind to the magnetic particles, and 200 µL of a substrate solution containing AMPPD (Lumipulse (registered trademark) substrate solution produced by FUJIREBIO Inc.) was added, and the amount of luminescence generated by enzyme reaction was counted as a signal amount. All the detecting step of the present Examples was conducted by using automated analysis instrument LUMIPULSE L2400 (registered trademark, manufactured by FUJIREBIO Inc.). Each labeling buffer was an aqueous solution containing components shown in Table 1 given below. A count value in each concentration of cyclosporin (ng/mL) is shown in Table 2 given below. Table 2 also shows a ratio between a count value when the concentration of cyclosporin was 100 ng/mL and a count value when the concentration of cyclosporin was 0 ng/mL (100(ng/mL)/0(ng/mL)), and a difference between the count values (100(ng/mL)-0(ng/mL)) (hereinafter, the same applies in Table 4 and Table 6.

**[Table 1]**

| | Comparative Example 1 | Example 1 | Example 2 | Example 3 |
|---|---|---|---|---|
| Imidazole | 0 mM | 5 mM | 15 mM | 20 mM |
| Tris | 50 mM | | | |
| ZnCl2 | 0.3 mM | | | |
| MgCl2 | 2.0 mM | | | |
| pH | 7.2 | | | |

**[Table 2]**

| | | Comparative Example 1 | Example 1 | Example 2 | Example 3 |
|---|---|---|---|---|---|
| Concent ration of cyclosporin [ng/mL] | 0 (*) | 4672 | 11326 | 6845 | 4583 |
| | 100 (*) | 25137 | 84343 | 59463 | 35536 |
| | 300 | 256116 | 734573 | 638649 | 445021 |
| | 800 | 3746812 | 6837463 | 7223307 | 6105039 |
| | 2000 | 24195534 | 30822761 | 34256566 | 31851274 |
| Ratio of * (100/0) | | 5.4 | 7.4 | 8.7 | 7.8 |
| Difference between * (100-0) | | 20465 | 73017 | 52618 | 30953 |

As shown in Table 2, it was confirmed that the detection sensitivity was significantly increased in the conditions in which imidazole was added (for example, Examples 1 to 3) even when the concentration of the test substance (cyclosporin) was low (for example, 100 ng/µL) as compared with the reaction system of cyclosporin and the labeling body, that is, the conditions in which the labeling buffer did not contain imidazole (for example, Comparative Example 1).

### (Examples 4 to 5 and Comparative Example 2)

20 µL of the cyclosporin solution prepared in the above-described (3) and 100 µL of a pretreatment liquid were mixed, and reacted at 37°C for 6.5 minutes. Subsequently, 50 µL of the magnetic particle solution containing the antibody-binding magnetic particles prepared in the above-described (1) was mixed, and reacted at 37°C for 8 minutes. The magnetic particles were magnetically collected and washed to remove a contaminant which did not bind to the magnetic particles. Subsequently, the ALP-labeled antibody prepared in the above-described (2) was added to 50 µL of a labeling body fluid diluted with a labeling buffer containing imidazole having a concentration of 15 mM (a concentration of the labeling body in the reaction system: 2 µg/mL), and reacted at 37°C for 8 minutes. After the reaction, the magnetic particles were magnetically collected and washed to remove a component which did not bind to the magnetic particles, and 200 µL of a substrate solution containing AMPPD (Lumipulse (registered trademark) substrate solution produced by FUJIREBIO Inc.) was added, and the amount of luminescence generated by enzyme reaction was counted as a signal amount. All the detecting step of the present examples was conducted by using automated analysis instrument Lumipulse L2400 (registered trademark, manufactured by FUJIREBIO Inc.). Each labeling buffer was an aqueous solution containing compositions shown in Table 3 given below. A count value in each concentration of cyclosporin (ng/mL) is shown in Table 4 given below.

**[Table 3]**

| | Comparative Example 2 | Example 4 | Example 5 |
|---|---|---|---|
| Imidazole | 15 mM | | |
| Tris | 50 mM | | |
| ZnCl2 | 0 mM | 0.1 mM | 0.3 mM |
| pH | 7.2 | | |

**[Table 4]**

| | | Comparative Example 2 | Example 4 | Example 5 |
|---|---|---|---|---|
| Concentration of cyclosporin [ng/mL] | 0(*) | 1246 | 1365 | 5708 |
| | 100(*) | 3543 | 4534 | 50486 |
| | 300 | 135214 | 134461 | 575101 |
| | 800 | 3745983 | 3779993 | 6385268 |
| | 2000 | 26077198 | 26087956 | 29698657 |
| Ratio of * (100/0) | | 2.8 | 3.3 | 8.8 |
| Difference between * (100-0) | | 2297 | 3169 | 44778 |

As shown in Table 4, it was confirmed that the detection sensitivity was significantly increased in the conditions in which a polyvalent metal ion was added (for example, Examples 4 to 5) even when the concentration of the test substance (cyclosporin) was low, as compared with the reaction system of cyclosporin and the labeling body, that is, the conditions in which the labeling buffer did not contain a polyvalent metal ion (zinc ion) (for example, Comparative Example 2).

### (Examples 6 to 7 and Comparative Example 3)

20 µL of the cyclosporin solution prepared in the above-described (3) and 100 µL of a pretreatment liquid were mixed and reacted at 37°C for 6.5 minutes. Subsequently, 50 µL of the magnetic particle solution containing the antibody-binding magnetic particles prepared in the above-described (1) was mixed, and reacted at 37°C for 8 minutes. The magnetic particles were magnetically collected and washed to remove a contaminant which did not bind to the magnetic particles. Subsequently, the ALP-labeled antibody prepared in the above-described (2) was added to 50 µL of a labeling body fluid diluted with a labeling buffer containing imidazole having a concentration of 15 mM (a concentration of the labeling body in the reaction system : 2 µg/mL), and reacted at 37°C for 8 minutes. After the reaction, the magnetic particles were magnetically collected and washed to remove a component which did not bind to the magnetic particles, and 200 µL of a substrate solution containing AMPPD (Lumipulse (registered trademark) substrate solution, produced by FUJIREBIO Inc.) was added, and the amount of luminescence generated by enzyme reaction was counted as a signal amount. All the detecting step of the present example was conducted by using automated analysis instrument Lumipulse L2400 (registered trademark, manufactured by FUJIREBIO Inc.). Each labeling buffer was an aqueous solution containing components shown in Table 5 given below. A count value in each concentration of cyclosporin (ng/mL) is shown in Table 6 given below.

**[Table 5]**

| | Comparative Example 3 | Example 6 | Example 7 |
|---|---|---|---|
| Imidazole | 15 mM | | |
| Tris | 50 mM | | |
| NiCl2 | 0 mM | 0.3 mM | 4.2 mM |
| pH | 7.2 | | |

**[Table 6]**

| | | Comparative Example 3 | Example 6 | Example 7 |
|---|---|---|---|---|
| Concentration of cyclosporin [ng/mL] | 0(*) | 1223 | 1278 | 1257 |
| | 100(*) | 3821 | 4419 | 5197 |
| | 300 | 142938 | 156371 | 171548 |
| | 800 | 4166821 | 4170081 | 3921631 |
| | 2000 | 28010360 | 26624190 | 22114223 |
| Ratio of * (100/0) | | 3.1 | 3.5 | 4.1 |
| Difference between * (100-0) | | 2598 | 3141 | 3940 |

As shown in Table 6, it was confirmed that the detection sensitivity was increased in the conditions in which a polyvalent metal ion was added (for example, Examples 6 to 7) even when the concentration of the test substance (cyclosporin) was particularly low, as compared with the reaction system of cyclosporin and the labeling body, that is, the conditions in which the labeling buffer did not contain a polyvalent metal ion (nickel ion) (for example, Comparative Example 3).

### [Industrial Applicability]

The present invention makes it possible to provide a detection method capable of detecting a test substance with high sensitivity by an immunological detection method, as well as a labeling buffer for use in the detection method, and a kit comprising the same.

## Claims

1. A method for immunologically detecting a test substance in a sample, comprising:
a labeling step of forming a complex of the test substance and a labeling body which contains a first probe molecule capable of binding to the test substance and a labeling substance in the presence of at least one selected from imidazole and a derivative thereof, a pH buffering agent, and a polyvalent metal ion.

2. The detection method according to claim 1, wherein a concentration of the at least one selected from imidazole and a derivative thereof is 1 mM or more in a reaction system of the labeling step in terms of imidazole.

3. The detection method according to claim 1, wherein a pH of a reaction system of the labeling step is 6.0 to 8.0.

4. The detection method according to claim 1, wherein a concentration of the polyvalent metal ion is 0.01 mM or more in a reaction system of the labeling step.

5. The detection method according to claim 1, further comprising a capturing step of capturing the test substance with a capturing body which contains a second probe molecule capable of binding to the test substance and an insoluble carrier.

6. The detection method according to claim 5, further comprising a washing step of removing a contaminant which has not been captured by the capturing body after the capturing step.

7. The detection method according to claim 1, wherein the labeling substance is an enzyme, and the first probe molecule is an antibody capable of binding to the test substance.

8. A labeling buffer for used in the detection method according to any one of claims 1 to 7, comprising at least one selected from imidazole and a derivative thereof, a pH buffering agent, and a polyvalent metal ion.

9. The buffer according to claim 8, further comprising a labeling body which contains a first probe molecule capable of binding to the test substance and a labeling substance.

10. A kit for used in the detection method according to any one of claims 1 to 7, comprising a labeling buffer which contains at least one selected from imidazole and a derivative thereof, a pH buffering agent, and a polyvalent metal ion.

11. The kit according to claim 10, further comprising a labeling body which contains a first probe molecule capable of binding to the test substance and a labeling substance.
